Europäisches Patentamt

(19) European Patent Office

Office européen des brevets

(11) Veröffentlichungsnummer. **0 113 040**
**A2**

(12) # EUROPÄISCHE PATENTANMELDUNG

(21) Anmeldenummer: 83111822.9

(22) Anmeldetag: 25.11.83

(51) Int. Cl.³: **C 08 B 37/10**

(30) Priorität: 30.11.82 DE 3244214

(43) Veröffentlichungstag der Anmeldung:
11.07.84 Patentblatt 84/28

(84) Benannte Vertragsstaaten:
AT BE CH DE FR GB IT LI LU NL SE

(71) Anmelder: Intermedicat GmbH
Gerliswilstrasse 45
CH-6020 Emmenbrücke(CH)

(72) Erfinder: Danigel, Karl Heinz
Gisberzstrasse 2
D-3508 Melsungen(DE)

(72) Erfinder: Feller, Wolfgang, Dr.
Ernstbergstrasse 39
D-3508 Melsungen(DE)

(74) Vertreter: von Kreisler, Alek, Dipl.-Chem. et al,
Deichmannhaus am Hauptbahnhof
D-5000 Köln 1(DE)

(54) Verfahren zur Reinigung und Fraktionierung von Heparin.

(57) Die Erfindung betrifft ein Verfahren zur Reinigung, Fraktionierung und Entpyrogenisierung von Heparin, seinen Salzen sowie synthetischen oder halbsynthetischen Heparinoiden, das dadurch gekennzeichnet ist, daß Heparin bzw. Heparinoide einem Ultrafiltrationsverfahren mit zwei Membranen unterworfen werden, wobei sie zunächst in einer ersten Stufe durch eine Ultrafiltrationsmembran mit einer nominalen Molekulargewichtsgrenze von 10 000 bis 50 000 permeieren und dann in einer zweiten Stufe von einer Ultrafiltrationsmembran mit einer nominalen Molekulargewichtsgrenze von 200 bis 5 000 zurückgehalten werden.

Durch zusätzliche Verwendung polarer organischer Lösungsmittel läßt sich die Molekulargewichtsgrenze der Ultrafiltrationsmembran mit höherer molekularer Durchlässigkeit noch exakter einstellen.

## Verfahren zur Reinigung und Fraktionierung
## von Heparin

Die Erfindung betrifft ein neues Trennverfahren zur Reinigung und Fraktionierung von Heparin, Heparin-ähnlichen Substanzen (Glycosaminoglykanen) und synthetisch oder halbsynthetisch hergestellten Heparinoiden sowie deren weitere Verwendung.

Heparin, das für die klinische Anwendung im prophylaktischen und therapeutischen Bereich eingesetzte gerinnungshemmende und antithrombotisch wirkende Glycosaminoglycan, besitzt eine komplexe, heterogene chemische Struktur. Wie andere Glycosaminoglykane ist es aus sich wiederholenden linear verknüpften, anionischen (Di-)saccharideinheiten aufgebaut, wobei in Heparin zu ca 70 % 1,4-verknüpfte L-Iduronsäure und D-Glucosamin gefunden werden. Iduronsäurereste sind in der 2-Stellung O-sulfatiert, der Glucosaminbaustein ist N-sulfatiert und in der 6-Stellung des Saccharidgerüstes O-sulfatiert. Außerdem werden noch ß-Glucuronsäure und 6-O-sulfatiertes N-Acetyl-$\alpha$-D-Glucosamin gefunden (B. Casu, Pharmacol.Res. Commun. $\underline{11}$, (1), (1979)).

Als Naturprodukt weist Heparin jedoch im Gegensatz zu Proteinen keine einheitliche Sequenz der Einzelbausteine auf, was durch die prinzipiell verschiedene Biosynthese bedingt ist.

Die Heterogenität dieser kommerziell erhältlichen Heparine zeigt sich unter anderem in schwankenden Gehalten der Elementarzusammensetzung wie auch in wechselnden Anteilen der Glucosamin- und Uronsäurereste. Handelsübliches Heparin hat normalerweise eine breite Molekulargewichtsverteilung von 4000 bis ca. 30 000 mit Molekulargewichtsmaximum von 12 - 15 000. Auch isoelektrische Fokussierung und elektrophoretische Methoden bestätigen, daß in Heparin eine Mischung sehr vieler verschieden langer, anionischer Einzelheparinketten vorliegt (E.A. Johnson und B. Mulloy, Carbohydrate Research 51, 119 (1976)).

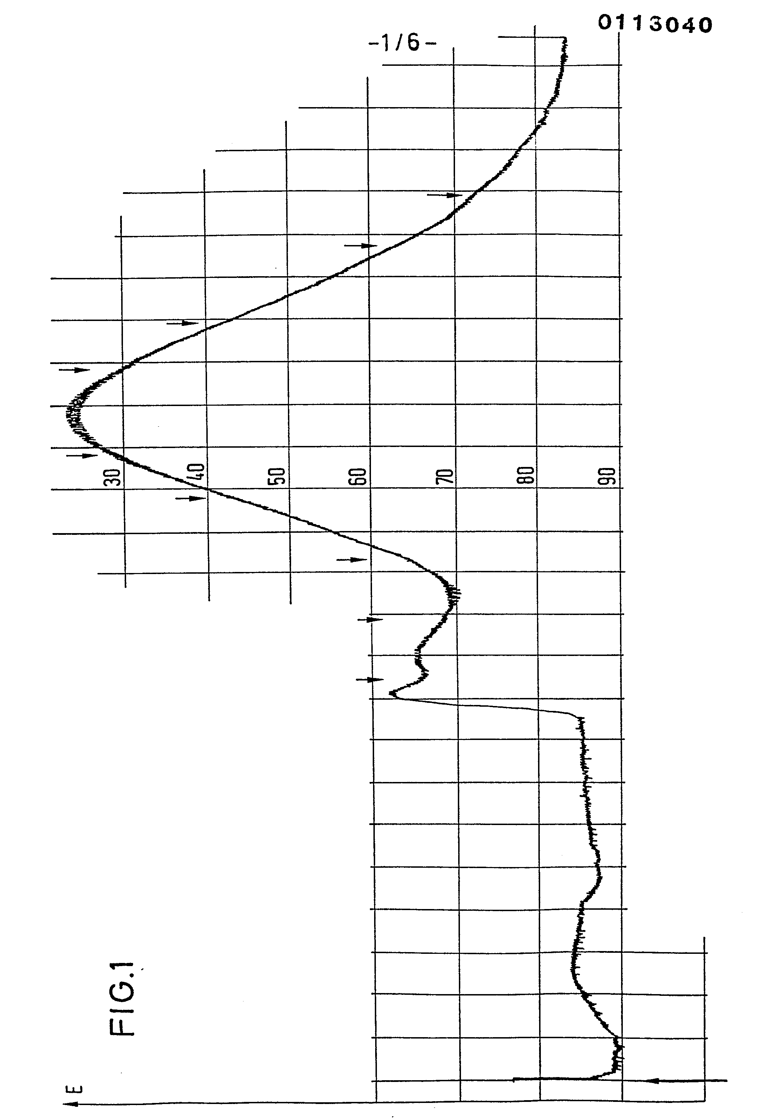

Im Zuge der Verfeinerung analytischer Methoden konnte darüber hinaus nachgewiesen werden, daß handelsübliches Heparin außerdem kleine, wechselnde Anteile anderer Glycosaminoglykane, z.B. Dermatan- und Heparansulfat, enthalten kann, die sich in ihrer biologischen Aktivität und ihrem Molekulargewicht von Heparin unterscheiden. Die Auftrennung eines Heparins durch Gelfiltration über eine Sephadex G-100$^R$ oder Sephadex G-50$^R$-Säule zeigt einige hoch- und niedermolekulare Verunreinigungen (Fig. 1).

Dieser Sachverhalt wird auch z.B. von R. Losito et al in J.Chromat 226, 61-67 (1981) beschrieben. Ein Verfahren zur Entfernung niedermolekularer Verunreinigungen in Heparinpräparationen wie Oxalaten, Carbonaten, Sulfaten und anderen Mineralsalzen wird beispielsweise in der GB-PS 1 602 439 beschrieben.

Als Verunreinigungen des Heparins sind außerdem geringe Anteile von Nucleinsäuren oder Nucleinsäurespaltprodukte, Proteine und Aminosäuren nachgewiesen worden.

Auch wenn die Gehalte der Verunreinigungen in den von den Pharmacopoen erlaubten Grenzen liegen, besteht trotzdem aus Gründen der Arzneimittelsicherheit ein Bedarf an einem Verfahren, das von Heparinsalzen sowohl die hochmolekularen, heparinähnlichen Verunreinigungen als auch gleichzeitig die niedermolekularen, meist salzartigen Verunreinigungen abtrennt.

Auch die Stabilität injektionsgerechter Heparinzubereitungen wird durch die Heparinreinheit beeinflußt.

Heparin ist in unterschiedlichen molekularen Formen in Geweben oder Einzelzellen nachgewiesen worden, wobei es frei und an Protein gebunden vorliegen kann. Klinisch verwendete Heparinsalze werden aus Lunge oder Intestinalschleimhaut von Schlachttieren durch Extraktion mit Kaliumacetat-, alkalischer Ammoniumsulfatlösung o.dgl. gewonnen und stellen stets Produkte mit minimaler Größe der natürlich in den Geweben vorkommenden zum Teil sehr großen Heparinmoleküle dar.

Ein Reinigungsverfahren sollte jedoch gleichzeitig in der Lage sein, abgesehen von der Abtrennung dieser hoch- und niedermolekularen Verunreinigungen zusätzlich auch noch den Heparinrohstoff in hoch- und niedermolekulare Fraktionen aufzutrennen.

Es sind verschiedene Verfahren zur Herstellung von Heparinfraktionen bekannt, deren Eigenschaften in der klinischen Anwendung dem herkömmlichen, unfraktionierten Heparin überlegen sind (V.V.Kakkar, British Medical Journal, Vol. 284, 375 - 379 (1982)).

Heparinspaltprodukte lassen sich chemisch oder enzymatisch nach in DE-OS 31 02 621, PCT/US81/00519 beschriebenen Verfahren herstellen. Heparinfraktionen unterschiedlichen Molekulargewichts hemmen je nach Kettenlänge und -zusammensetzung die Blutgerinnung unterschiedlich stark oder steigern sogar die Blutung und geben dadurch Anlaß zu Blutungsrisiken. Der Zusammenhang zwischen durchschnittlichem Molekulargewicht und molekularem Aufbau des Heparins einerseits und seinen antithrombotischen bzw. hämorrhagischen Eigenschaften andererseits ist noch nicht bis
ins Detail bekannt, doch erscheint es wünschenswert,
Heparine in Fraktionen mit enger Molekulargewichtsverteilung zu erhalten, um den Koagulationsmechanismus
gezielt beeinflussen zu können.

In der klinischen Praxis genutzte Effekte des Heparins
beruhen jedoch in erster Linie auf seiner antithrombotischen, antikoagulatorischen oder antilipämischen Aktivität.

Verfahren zur Herstellung solcher Fraktionen sind:
- chemische Spaltung des Heparinmoleküls in kleinere
  Bruchstücke, wobei oft chemische Schritte zur Wiedereinführung von Sulfatresten vorgenommen werden
  müssen,
- fraktionierte Fällungsverfahren z.B. mit Ethanol,
- Affinitätschromatographische Verfahren, z.B. an immobilisiertem Antithrombin III,
- Gelfiltration, beispielsweise über Sephadex G-100[R],
- Elektrophoretische Verfahren, isoelektrische Fokussierung,
- Chromatographie an Ionenaustauschern und hydrophobe
  Chromatographie,
- Ultrafiltrationsverfahren
- Ausschüttelverfahren
- Ultrazentrifugation im Dichtegradienten,
- Kombinationen einzelner oben genannter Verfahren.

Abgesehen von den chemischen Spaltungen nutzen die
Fraktionierungsverfahren einerseits Unterschiede in
der chemischen Struktur oder in physikalischen Eigenschaften wie Ladung, Ladungsdichte, Kettenlänge oder
davon abhängige Eigenschaften wie Löslichkeitsunterschiede und andererseits Differenzen der Bindungsaffinität einzelner Heparinketten zu Plasmaproteinen wie Antithrombin III aus.

Diese zum Teil bisher nur in analytischem Maßstab angewandten Verfahren weisen jedoch den Nachteil auf, daß entweder ihre Ausbeute sehr gering ist, oder ihre Trennschärfe nicht ausreicht, obwohl Produkte mit hoher biologischer Gerinnungsaktivität erhalten wurden. Andererseits sind einige Verfahren besonders unter wirtschaftlichen Gesichtspunkten nicht praktikabel wie etwa die Gelfiltration, deren Trennschärfe bei genügend langen Chromatographiesäulen durchaus befriedigende Ergebnisse liefert.

Ein weiterer - ebenfalls unter fertigungstechnischen und wirtschaftlichen Aspekten bedeutsamer und bisher unbeachteter - Aspekt ist, daß bei nahezu allen Trennverfahren das gewünschte Produkt nur in geringen Konzentrationen gewonnen wird. Es fallen deshalb große Flüssigkeitsvolumen mit relativ kleinen Produktmengen an, die erst in aufwendigen arbeits-, material- und zeitintensiven Folgeschritten aufzuarbeiten sind. Naturgemäß sinkt damit auch die Ausbeute des gesamten Trenn- und Aufarbeitungsverfahrens, zumal - wie Versuche zeigten - das wertvolle fraktionierte Heparin besonders aus verdünnten Lösungen nur zu etwa 80% wiederzugewinnen ist. Ein solcher Schritt ist beispielsweise die Ausfällung der Heparinsalze mit Ethanol oder Aceton in hohen Konzentrationen ( $\gtrsim$ 80%), so daß sehr viel Lösungsmittel zur Ausfällung kleiner Produktmengen benötigt wird und sich dadurch der Gesamtprozeß stark verteuert. Hinzu kommt, daß noch mindestens ein Umfällungsschritt anzuschließen ist, um das Produkt salzfrei zu erhalten. Erst daran anschließend erfolgt die Abtrennung des Heparinpräzipitats von der Mutterlauge und ein Trocknungsprozeß.

Diese diskontinuierlich aufeinanderfolgenden Arbeitsschritte zur Konzentrierung verhindern zudem weitgehend Automatisierungsmöglichkeiten. Eine Beispielrechnung der Heparinfraktionierung illustriert das Problem:

Bei der Gelfiltration von 280 g in 1,1 l Eluent gelöstem Heparin-Natrium über eine 90 cm lange Sephadex G-100$^R$-Säule (K S 370 der Fa. Pharmacia) mit einem Säulenvolumen von 96 l, fallen 90 g des gewünschten niedermolekularen fraktionierten Produktes in 20 l Eluent an. Die durchschnittliche Heparinkonzentration des Produkts liegt bei 0,45 %.
Zur vollständigen Ausfällung dieser 90 g Produkt mit Ethanolendkonzentration 80 % (V/V) werden 80 l Ethanol benötigt.

Ein weiteres Konzentrierungsverfahren besteht darin, die verdünnten Heparinlösungen an Anionenaustauschern, z.B. Lewatit$^R$ oder Dowex $^R$ zu adsorbieren und anschließend wieder unter geeigneten Bedingungen durch pH- und/oder Ionenstärke-Änderung zu desorbieren.

Getestet wurde das Adsorptionsvermögen der Materialien für Heparinfraktionen und die Ausbeute, mit der Heparin-Natrium wieder eluierbar ist. Dabei ist noch zu beachten, daß solche Elutionsbedingungen zu vermeiden sind, die Fremdionen einschleppen (z.B. Ca$^{2+}$) oder Heparin chemisch verändern ( Hydrolyse). Als Ergebnis derartiger Tests zeigte sich, daß die Kapazität der Austauscher für einen wirtschaftlichen Einsatz zu niedrig war.

Die Ausbeute der Elution des gebundenen Heparins ist unzureichend (50 - 60%), wenn die Heparinfraktion nur in kleinen Volumina - also konzentriert - von den Austauschern eluiert werden soll. Die Ausbeute steigt, wenn akzeptiert wird, daß große Elutionsvolumina verwendet werden. Ohne Erfolg blieb auch die Variation der Elutionsbedingungen hinsichtlich isokratischer/-Gradientenelution, Ionenstärke, pH-Wert.

Obwohl der Einsatz der oben genannten Ionenaustauscher für die Heparinisolierung von ungereinigten Präparationen beschrieben ist, versagt das Verfahren dann, wenn die schonende Elution des adsorbierten Heparins mit kleinen Volumina des Elutionspuffers als Konzentrierungsschritt erforderlich wird. Auch an diesen nicht gangbaren Elutionsschritt müßte jedoch noch wiederum ein Fällungsschritt angeschlossen werden.

Aus Obigem folgt, daß der Gesamtaufwand eines Reinigungs- und Fraktionierungsverfahrens nicht nur von dem Aufwand des eigentlichen Trennungs/Fraktionierungsschritts abhängig ist, sondern sehr stark von dem sich daran anschließenden Konzentrierungsverfahren beeinflußt wird.

Ein bisher nicht beachteter Kostenfaktor besteht darin, ein gereinigtes/fraktioniertes Heparinsalz zunächst als lagerfähige Festsubstanz zu isolieren, später wieder aufzulösen und in eine injektionsgerechte Form zu bringen, statt Heparin bzw. seine Salze in geeignet konzentrierten Lösungen direkt im Anschluß an den Reinigungs-/Fraktionierungsprozeß zur Abfüllung zu bringen.

Ultrafiltrationsverfahren heparinhaltiger Lösungen sind aus den DE-OSen 29 45 595 und 31 06 876 bekannt.

Ultrafiltrationen solcher Lösungen werden außerdem im Rahmen der Lipoproteinanalytik bei Humanplasma angewendet, das zuvor mit Heparin und Mangansalzen behandelt wurde (G. Russel-Warnick, John J. Albers Clin.

Chem. 24/6, 900 - 904 (1978)).

Nachteilig bei den Verfahren ist, daß zum Teil bei sauren pH-Werten, vorzugsweise pH 1 bis pH 5,5, gearbeitet werden muß. Die Einhaltung des stets sauren pH-Wertes dient, wie in der DE-OS 29 45 595 beschrieben, dazu, bei Verwendung einer gegebenen Membran das Molekulargewicht des Produktes zu steuern. Saure pH-Werte sind jedoch wegen der begrenzten Heparinstabilität nachteilig. Insbesondere dann, wenn über viele Stunden hin ultrafiltriert wird, ist verstärkt mit hydrolytischem Abbau und Inaktivierung des Heparins zu rechnen. Der erstrebenswerte pH-Bereich der zu ultrafiltrierenden Lösung sollte deshalb bei pH 6,0 - 8,0 liegen.

Es ist außerdem bekannt, Infusions- und Injektionslösungen mittels Ultrafiltrationsverfahren durch Membranen mit nominaler Molekulargewichtsgrenze von 10 000 von Pyrogenen zu befreien (G. Koppensteiner et al, Pharm.Ind. 38, 19, 827-931 (1976)). Dabei werden jedoch stets Lösungen mit niedermolekularen Wirk- bzw. Inhaltsstoffen behandelt, die ungehindert durch die Ultrafiltrationsmembran durchtreten können. Nicht anzuwenden ist dieses Verfahren selbstverständlich dann, wenn sowohl die Pyrogene als auch die Wirksubstanzen von der Membran zurückgehalten werden. Eine Membran mit Molekulargewichtsgrenze 10 000 läßt nur den niedermolekularen Teil von Heparin durchtreten, so daß im pyrogenfreien Ultrafiltrat nur eine niedermolekulare Heparinfraktion gefunden wird. Die Ausbeute des Verfahrens ist deshalb nur gering. Außerdem ist das Ultrafiltrat gegenüber der Ausgangslösung stark verdünnt, so daß das Verfahren zur Entpyrogenisierung von Heparin im industriellen Maßstab ausscheidet.

Es besteht Bedarf an einem wirtschaftlichen, kontinuierliche Arbeitsweise erlaubenden, wenig arbeitsintensivem Trenn- und Reinigungsverfahren, das es ermöglicht, unter keimarmen Bedingungen von den oben genannten Begleitstoffen abzutrennen oder wahlweise zu fraktionieren und gleichzeitig anzukonzentrieren. Dabei sollte das Produkt ohne Ausfällungsschritte unmittelbar nach dem Trenn/Reinigungsschritt in die gewünschte injektionsgerechte Konzentration und unverzüglich zur Abfüllung gebracht werden können.

Die vorliegende Erfindung betrifft nun ein Verfahren zur Reinigung, Fraktionierung und gleichzeitigen Entpyrogenisierung von Heparin, seinen Salzen und/oder seinen Derivaten mit Hilfe der Ultrafiltration, das dadurch gekennzeichnet ist, daß man wäßrige Lösungen des Heparins bzw. seiner Salze einem Ultrafiltrations-Zweimembran-Verfahren unterwirft, wobei Heparin und seine Derivate zunächst in einer ersten Stufe durch eine Ultrafiltrationsmembran I mit relativ hoher molekularer Durchlässigkeit permeieren und erst dann in einer zweiten Stufe der Filtration von einer Ultrafiltrationsmembran II mit relativ geringer molekularer Durchlässigkeit zurückgehalten werden.
Es ist auch möglich, daß nur ein Teil des Heparins permeiert, und zwar nur derjenige Teil, der zu erhalten gewünscht wird.

Das erfindungsgemäße Verfahren zur Reinigung und Fraktionierung von Heparin sieht die Verwendung mindestens zweier Ultrafiltrationsmembranen mit unterschiedlichen Trennbereichen vor, die zu folgenden Vorteilen führen:

Es wird nur mit kleinen Flüssigkeitsvolumen gearbeitet.

Das Gesamtverfahren weist kontinuierlich ineinander übergehende, sehr wenig arbeitsintensive Arbeitsschritte auf und ist automatisierbar.

Die Abtrennung von hochmolekularen und niedermolekularen Verunreinigungen ist möglich. Gleichzeitig kann das Verfahren je nach Wahl der Arbeitsbedingungen auch zur Fraktionierung des Heparins eingesetzt werden. Das Molekulargewicht des Produktes ist in engen Grenzen steuerbar. Das Verfahren erlaubt unter keimarmen Bedingungen zu arbeiten. Das Produkt kann direkt in eine injektionsgerechte Form abgefüllt werden.

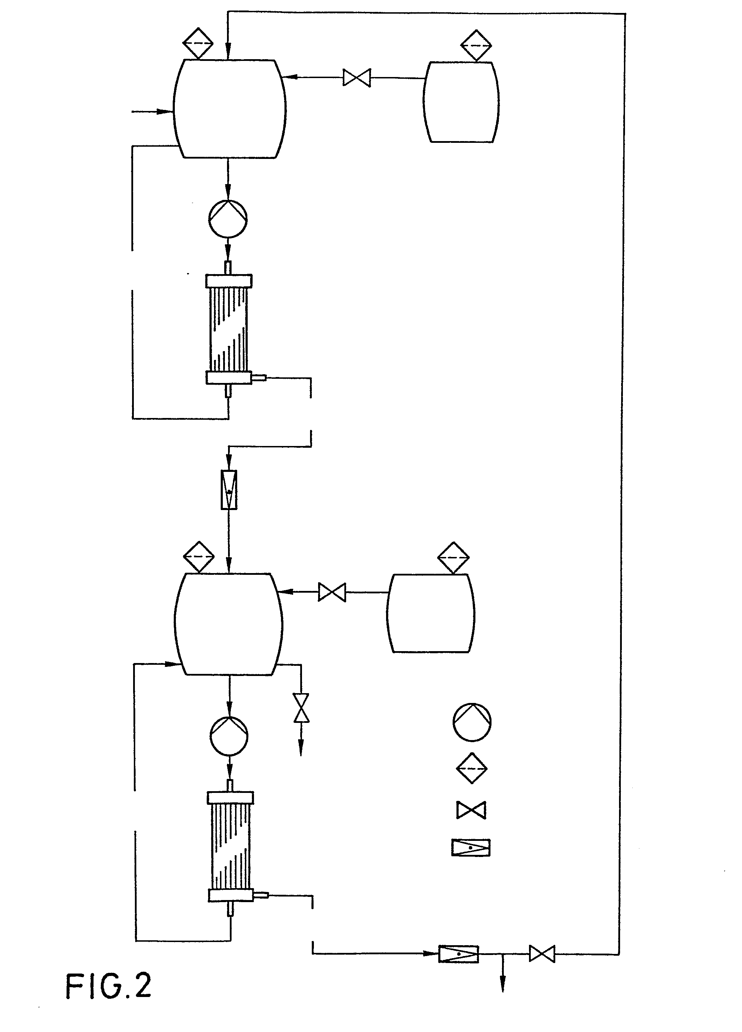

Das erfindungsgemäße Zweimembranverfahren wird im Folgenden anhand der Fig. 2 beschrieben.

Das Heparin wird durch eine erste Ultrafiltrationsmembran I ultrafiltriert, so daß ein Produkt der gewünschten Reinheit bzw. Molekulargewichtsverteilung unter Abtrennung von hochmolekularen Fremd- und/oder Heparinanteilen durch die Membran durchtritt. Das erhaltene Ultrafiltrat I enthält Heparin nur in geringen Konzentrationen.

Die Anreicherung erfolgt über eine zweite Ultrafiltrationsmembran II, deren Trenncharakter so ausgelegt ist, daß Heparin nicht oder nur sehr wenig permeiert, dagegen Salze und Wasser durchtreten.

Da sich infolge des Flüssigkeitsentzugs die Heparinkonzentration in der Ansatzlösung ständig erhöhen würde, was zur Viskositätserhöhung und zu vermindertem Ultrafiltratfluß durch die Membran I führen würde, muß das Ansatzvolumen konstant gehalten werden. Dies geschieht dadurch, daß zunächst in der Anlaufphase des Trennungsprozesses Salzlösung aus einem Vorratsgefäß eingespeist wird, und darauf folgend - wenn sich im Ultrafiltrat I (Produktreservoir) ein genügend großes Flüssigkeitsreservoir angesammelt hat - das Ultrafiltrat II
wieder in die Ansatzlösung zurückgeleitet wird.

Die Vorteile dieser erfindungsgemäßen Anordnung sind
dadurch gegeben, daß
die Trennung im Flüssigkeitskreislauf ohne Verbrauch
von Lösungen (preiswert) durchführbar ist, kein Verlust an Heparin entsteht und man eine ständige Produktsanreicherung im Produktreservoir erreicht.

Der Heparinansatzlösung müssen Salze zur Erhöhung der
Ionenstärke, vorzugsweise Kochsalz, beigegeben werden.
Diese lassen sich aus dem Produktreservoir leicht zusammen mit den salzartigen, niedermolekularen Verunreinigungen dadurch entfernen, daß der erste Trenn- oder
Fraktionsteil (Membran I) der Anlage stillgelegt und
die produkthaltige Salzlösung im Produktreservoir gegen
Wasser ausgetauscht wird (Diafiltration). Aufgrund der
geringen Durchlässigkeit der Membran II für Heparin
besteht (nahezu) kein Verlust an Heparin. Dadurch, daß
die Möglichkeit besteht, den Flüssigkeitszufluß durch
das Gefäß mit dem Ultrafiltrat I oder den Zufluß von
Wasser zu dem Produktreservoir zu verhindern, läßt
sich die Heparinlösung im Laufe der Zeit nahezu beliebig (bis ca. 25 %) anreichern. Das gesamte System (Edelstahlleitungen, Pumpen) ist chemisch durch übliche
Maßnahmen ($H_2O_2$) sterilisierbar. Der Flüssigkeitszulauf erfolgt über Sterilfilter. Das gesamte Trenn- und
Leitungssystem ist geschlossen und nur über Sterilfilter
zu entlüften.

Es wurde nun weiterhin ein einfacher Weg gefunden,
Membranen mit zu kleiner Porenverteilung zu "erweitern",
d.h. das Durchtrittsverhalten von Heparin durch die Trennmembran I steuernd zu beeinflussen. Dies ist im Hinblick auf die nicht bekannte Reproduzierbakeit der jeweils vorliegenden Membran von hoher Bedeutung, um jeweils identische Produkte herstellen zu können. Durch geringe Zusätze wasserlöslicher organischer Lösungsmittel wie z.B. Isopropanol zu der Heparinlösung wird der Anteil Heparin, der die Trennmembran I durchtritt, vergrößert, d.h. das mittlere Molekulargewicht des Produktes zu größeren Werten verschoben. Zwar sinkt die Ultrafiltrationsgeschwindigkeit infolge der höheren Viskosität in Gegenwart des Alkohols etwas ab, dieser Nachteil kann jedoch durch die Möglichkeit, das Molekulargewicht des Produktes einstellen zu können, akzeptiert werden und wirkt sich lediglich in einer längeren Trennzeit aus.

Durch diese Maßnahme der Zugabe wasserlöslicher organischer Lösungsmittel kann die Molekulargewichtsverteilung des Produkts, d.h. die "Schnittstelle" mit der die Membran I den niedermolekularen Anteil des Ausgangsheparins abtrennt, auch bei nicht exakt "passenden" Membranen den Erfordernissen angepaßt werden. Die genaue Lösungsmittelkonzentration ist im Einzelfall experimentell, z.B. mittels Hochdruckflüssigkeitschromatographie, zu ermitteln und hängt von dem verwendeten Heparinrohstoff, dem pH-Wert und der Ionenstärke des Trennmediums sowie der gewünschten Molekulargewichtsverschiebung ab.

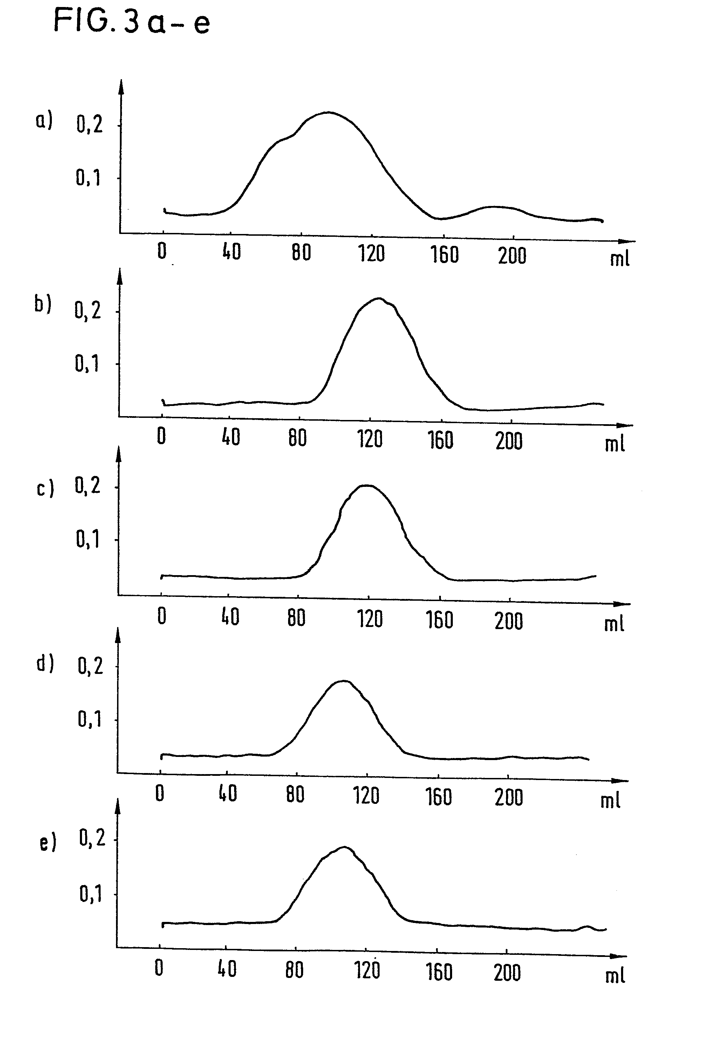

In Fig. 3 ist die Veränderung der Molekulargewichtsverteilung des im Produktreservoir gewonnenen Heparins durch Isopropanol gezeigt. Aufgetragen sind Elutionsdiagramme einiger Trennungen von Heparin über eine Sephadex G-100$^R$- Säule, wobei die Extinktion (bei 205 nm) des Säuleneluats gegen das Elutionsvolumen aufgetragen ist.

0113040

Fig. 3a  Elutionsprofil des unfraktionierten Ausgangsheparins, pH 7,0

Fig. 3b  Elutionsprofil einer durch eine Amicon H1-P
30-Ultrafiltrationsmembran von dem Heparin
(3a) abgetrennten Fraktion.
Ultrafiltrationsmedium: 5 % Heparin-Natrium
300 mM NaCl
kein Zusatz.

Fig. 3c  wie 3b, jedoch Ultrafiltrationsmedium verändert:
5 % Heparin-Natrium
300 mM NaCl
2 % Isopropanol

Fig. 3d  wie 3b und 3c, jedoch Ultrafiltrationsmedium
verändert:
5 % Heparin-Natrium
300 mM NaCl
7,5 % Isopropanol

Fig. 3e  wie 3a - d, jedoch Ultrafiltrationsmedium
verändert:
5 % Heparin
300 mM NaCl
15 % Isopropanol

In Fig. 3b - 3e ist die Verschiebung der Molekulargewichtsverteilung der gewonnenen Heparine nach links, zu höheren Molekulargewichten, deutlich erkennbar. Die Erhöhung von 7,5 auf 15 % Alkoholgehalt bewirkt in diesem Falle keine weitere Veränderung des Elutionsprofils.

Der Vorteil des Zweimembranverfahrens wird nachstehend verdeutlicht:

Bei Trennung von 500 g Heparin-Natrium über eine Amicon H5 P50-43-Membran fallen während 16stündiger Trennung insgesamt 80 l Ultrafiltrat mit 250 g niedermolekularer Heparinfraktion an, wenn der Konzentrierungsschritt über die Membran II (Amicon H5 P1) ausgeschaltet bleibt. Bei Anwendung des erfindungsgemäßen Verfahrens mit Konzentrierungsschritt liegt das Gesamtflüssigkeitsvolumen im Produktreservoirbei 7,5 l (Beispiel 1). Die Endkonzentration von 3,3 % Heparin kann noch durch weiteres Einengen oder Wasserzugabe verändert werden.

Zur Ausfällung des Heparins nach der Trennung sind 30 Liter Alkohol bei Verwendung des erfindungsgemäßen Verfahrens notwendig, während 240 l bei Ausfällung nach dem herkömmlichen Verfahren ohne Konzentrierungsschritt zugegeben werden müssen.

In einer vorteilhaften Ausführungsform des geschilderten Verfahrens kann man in Anwesenheit hoher Konzentrationen organischer Lösungsmittel arbeiten,ohne Heparinausfällung zu beobachten, wenn Heparinsalze mit organischen Kationen zur Trennung eingesetzt werden. Man beobachtet dabei ein unverändertes Fraktionierungsverhalten des Heparins, wobei reproduzierbar Fraktionen sehr eng geschnittener Molekulargewichtsverteilung erhalten werden können.

Als Kationen sind beispielsweise Monoalkyl-, Dialkyl-, Trialkyl- oder Tetraalkylammoniumsalze oder substituierte Arylammoniumsalze brauchbar. Die Trennung der organischen Heparinsalze wird auch hier lediglich durch die Lösungsmittelstabilität der Membran limitiert.

Durch Variation der verwendeten Membranen, der Konzentration der Zusatzstoffe in der Ansatzlösung wie Kochsalz,

dem pH-Wert in den Grenzen 6,0 - 8,0, der verwendeten Heparinsalze sowie des Zusatzes des wasserlöslichen organischen Lösungsmittels, ist es möglich, Heparine geeigneter Reinheit bzw. Molekulargewichtsverteilung mit dem geschilderten Zweimembranverfahren herzustellen. Es zeigt sich dabei, daß die Ultrafiltrationsgeschwindigkeit stark zunimmt, wenn der zu trennenden oder zu reinigenden Heparinlösung Kochsalz mit einer Endkonzentration von 50 mM bis 3 M beigesetzt wird. Die Reproduzierbarkeit des Ultrafiltrationsprozesses von Charge zu Charge ist nur dann einzuhalten, wenn die Ionenstärke der zu trennenden/reinigenden Heparinlösung durch Neutralsalz (Kochsalz) auf wenigstens 100 mM, vorzugsweise 250 mM konstant eingestellt wird. Das erfindungsgemäße Trennungs- und Reinigungsverfahren bedingt stets die Anwesenheit einer genügend hohen Salzkonzentration.

Durch die weite Variationsmöglichkeit der Trennbedingungen nach dem erfindungsgemäßem Verfahren können auch andere polyanionische, polysulfatierte, im weitesten Sinne Heparinähnlichkeit aufweisende Substanzen mit breiter Molekulargewichtsverteilung wie Chondroitinsulfate, Heparansulfat, Heparitinsulfat, Keratansulfat, Dermatansulfat, Hyaluronsäure oder auch Heparinanaloga ("Heparinoide") gereinigt und fraktioniert werden. Dabei seien Heparinoide definiert als Substanzen, die Wirkungen, insbesondere gerinnungshemmende Wirkungen, auslösen, die denjenigen des Heparins ähneln. Während sich diese Substanzen somit in ihrer Wirkung nahestehen, sind sie der chemischen Struktur nach Glykosaminoglykane, die in unterschiedlichem Umfang auch im menschlichen Organismus vorkommen. Es können aber auch Naturstoffe, deren chemische Struktur bei der Herstellung modifziert wird, Grundprodukte für halbsynthetische Heparinoide sein, und schließlich werden in neuerer Zeit auch zunehmend vollsynthetische Heparinanaloga entwickelt, die mit Heparin chemisch keine Gemeinsamkeit haben. (aus: U. Schmitz-Hübner "Heparinoide: Therapeutische Alternativen zu Heparin?" (S. 210 ff) in: "Hämostase, Thrombophilie und Arteriosklerose", herausg. J.van de Loo und F. Asbeck, F.K. Schattauer Verlag (1982)).

Heparinoide oder Heparinanaloga in diesem Sinne sind etwa Dextransulfate, sulfatierte Polysaccharide oder sulfatierte Pectinderivate, Cellulosesulfate und Pentosan oder Xylan-Polysulfate.

Das erfindungsgemäße Verfahren ist auch zur Reinigung und weiteren Fraktionierung von chemisch verändertem Heparin (Heparinderivate) oder Heparinspaltprodukten anwendbar, um hoch- oder niedermolekulare Bestandteile abzutrennen.

Die Möglichkeit, gemäß der vorliegenden Erfindung die Molekulargewichtsverteilung des durch eine gegebene Membran ultrafiltrierten Heparins unter Zusatz wasserlöslicher organischer Lösungsmittel zu verschieben, wird durch die von den Membranherstellern angegebene Lösungsmittelkompatibilität der im Handel erhältlichen Ultrafiltrationsmembranen eingeschränkt. So kommen erfindungsgemäß für Polysulfonmembranen folgende Lösungsmittel oder deren Mischungen in Betracht:
Butanol (70%), Ethanol (25%), 2-Ethoxyethanol (1%), Isopropanol (25 %), Diethanolamin.

Für Membranen auf Celluloseacetatbasis (Firma Sartorius, Göttingen) sind beispielsweise folgende Lösungsmittel oder Mischungen im Sinne der Erfindung verwendbar:

Methanol 98 %, Ethanol 98 %, Isopropanol, n-Propanol, Amylalkohol, Butanol.

Es sind durchaus auch andere Lösungsmittel, wie beispielsweise Tetrahydrofuran, Dimethylsulfoxid, Dimethylformamid, Acetonitril u.dgl. anwendbar, unter der Voraussetzung, daß die Trennmembran durch diese Lösungsmittel nicht dauerhaft geschädigt wird.

Im Rahmen des oben beschriebenen Verfahrens ist es außerdem möglich, Pyrogene abzutrennen, wenn man unter Verwendung des erfindungsgemäßen Zweimembranverfahrens mit einer Ultrafiltrationsmembran I mit nominaler Ausschlußgrenze von 10 000 bis 50 000 arbeitet und ggfls. zur Verschiebung des mittleren Molekulargewichts von Heparin im Produkt organische, wasserlösliche Lösungsmittel, vorzugsweise Isopropanol, zusetzt. Die Lösungen enthalten außerdem zur Erhöhung der Ionenstärke ein physiologisch verträgliches Neutralsalz wie NaCl. Die nominale Ausschlußgrenze von Membran II wird so gewählt, daß kein Heparin durchtritt und liegt im Molekulargewichtsbereich um 1 000. Die Ausbeute des Heparins im Produktreservoir wird durch Zugabe des wasserlöslichen organischen Lösungsmittels vergrößert.

Die Erfindung wird durch die nachfolgenden Beispiele näher erläutert.

Beispiel 1

500 g handelsübliches Heparin-Natrium mit einem mittleren Molekulargewicht von etwa 15 000 wurden in 10 l einer 250 mM Kochsalzlösung gelöst, der pH-Wert mit 1 N NaOH auf pH 7,0 korrigiert und diese Lösung durch ein 0,2 µm Membranfilter (Sartorius SM 11107) sterilfiltriert.

Diese Ansatzlösung wurde in das Ultrafiltrationstrennsystem gemäß Fig. 2 eingespeist. Das Trennsystem enthielt folgende Ultrafiltrationsmembranen:

Membran I:   Amicon Hohlfaserkapsel H5 P50-43 mit nominaler Molekulargewichtsgrenze 50 000 Membran II: Amicon Hohlfaserkapsel H5 P1-43 mit nominaler Molekulargewichtsgrenze 1 000

Die Temperatur aller Lösungen wurde bei 20°C gehalten.

In Fig. 2 ist das Flußdiagramm der Trennanordnung gezeigt.

Pumpe I und II: Hygietta 10 (Fa. Hilge GmbH., Bodenheim).

Alle Ansatzbehälter, Leitungen, Pumpen etc. waren nach bekannten Verfahren sterilisiert. Das geschlossene Trennsystem war über Sterilfilter entlüftet. Die Heparinlösungen wurden mit 300 l/h umgewälzt. Der Ultrafiltratfluß durch Membran I lag zu Beginn der Trennung bei 11 l/h. Eingangsdruck 0,69 bar, Ausgangsdruck 0,68 bar.

Das Ultrafiltrat I wurde im Produktreservoir aufgefangen. Der Flüssigkeitsverlust im Ansatzbehälter wurde durch entsprechenden Zufluß aus der "Vorratslösung" mit 0,25 M NaCl solange ausgeglichen, bis sich wenigstens 7,5 l Ultrafiltrat I angesammelt hatten. Erst dann wurde der zweite Ultrafiltratkreislauf mit der Konzentrierungsmembran II in Gang gesetzt. Das Ultrafiltrat II wurde in die Heparinansatzlösung zurückgeführt. Im weiteren Verlauf des Betriebes wurden die Volumina der Heparinansatzlösung und des Produktreservoirs dadurch konstant gehalten, daß jeweils die Eingangsdrucke der Membranen I und II verändert wurden.

Die Trennung im Ultrafiltrationskreislauf I wurde nach 16 Stunden abgebrochen und der Kreislauf II allein als Diafiltration weiter betrieben, wobei anstatt des Ultra-filtrats I jetzt $H_2O$ in das Produktreservoir einge-speist wurde. Das Ultrafiltrat II, das als Hauptbestandteil NaCl und die niedermolekularen Heparinverunreinigungen enthielt, wurde verworfen. Die Diafiltration wurde abgebrochen, als im Ultrafiltrat II kein Chlorid mehr nachweisbar war. Die Heparinkonzentration im Produktreservoir lag bei 3,33 %. Das Gesamtvolumen betrug 7,5 1. Nach einer Kontrolle der Heparinaktivität und Heparinkonzentration nach bekannten Verfahren wurde die Lösung durch weiteres Einengen auf 5 000 USP-Einheiten/0,5 ml entsprechend 10 % (w/w) Heparin über die Membran II eingestellt, die Gerinnungsaktivität nochmals überprüft und über einen 0,2 µm Membranfilter filtriert. Diese Lösung wurde - ohne Ausfällungsschritt - sofort in 1 ml- Brechampullen abgefüllt.

Kontrollversuche zeigten, daß die abgefüllte Heparinlösung sowohl keim- als auch pyrogenfrei war.

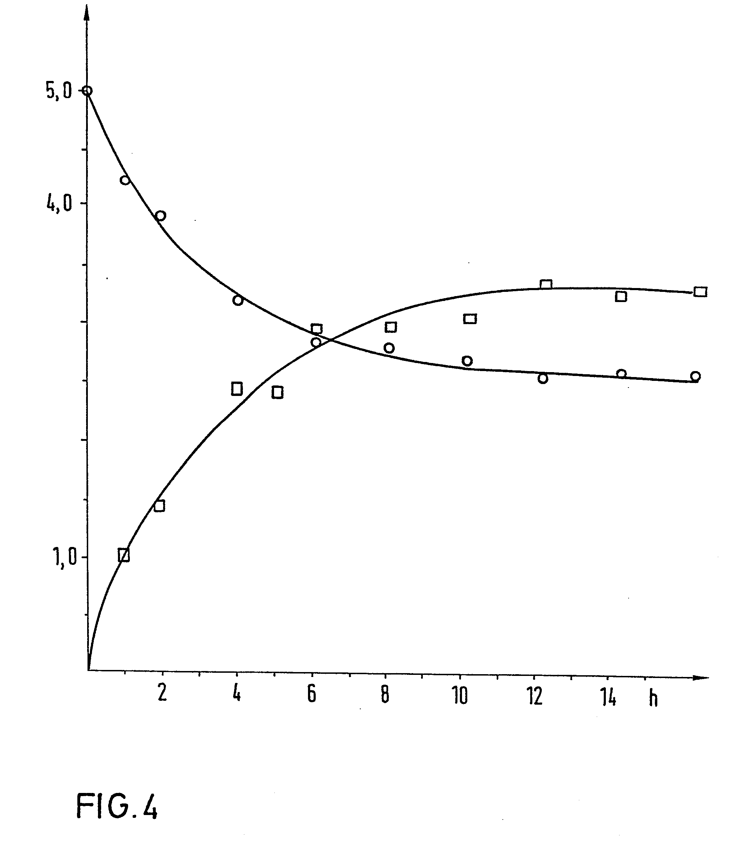

Der Konzentrationsverlauf des Heparinproduktes in der Heparinsalzlösung und im Produktreservoir ist in Fig. 4 graphisch dargestellt. Die Heparinkonzentration wird mit einem photometrischen Test mit Toluidinblau oder Azur A ermittelt (Lam, Silbert, Biochem. Biophys. Res. Comm. 69, 570-574 (1976)).

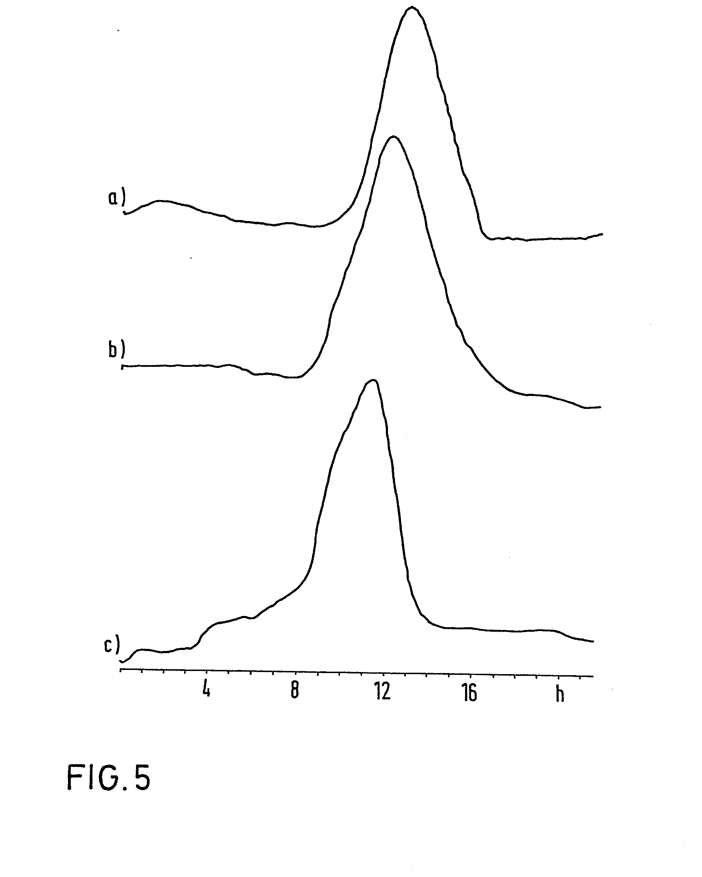

Fig. 5 zeigt die Elutionsdiagramme von Trennungen des Heparin-Ausgangsmaterials, des Retentats und des im Produktreservoir gewonnenen, gereinigten Heparins über eine (1,5 x 100 cm) Sephadex G-100[R]-Säule. Registriert ist die Extinktion bei 205 nm. Die im Ausgangsmaterial enthaltene niedermolekularen (salzartigen) und hochmolekularen Verunreinigungen wurden abgetrennt.

Die chemische Analyse des gereinigten Heparins (G) in Vergleich zu dem Ausgangsmaterial (A) ergab:

1. Elementarzusammensetzun

A: C 24,54 %, H 3,65 %, N 2,13 %, S 10,87 % G: C 24,06 %, H 3,17 %, N 2,14 %, S 10,88 %.

2. Gehalt freier Aminosäuren:

A: 9,04 μ mole/100 mg

G: unter der Nachweisgrenze ( 0,5 μ mole/100 mg)

3. Gehalt an Sulfat, Oxalat, Carbonat, Chlorid

A: Gesamt: 0,3 % G: Gesamt 0,05 % Beide Proben sind chloridfrei.

4. Gehalt an Phosphat

A: 0,1 % G: nicht nachweisbar

Beispiel 2

Durchführung mit einer Anordnung wie in Beispiel 1 beschrieben, jedoch wurden folgende Membranen eingesetzt:

Membran I:  Amicon H1P 30-43 mit nominaler Ausschlußgrenze bei Molekulargewicht 30 000

Membran II: Amicon H1 P1-43 mit Ausschlußgrenze bei
            Molekulargewicht 1 000.

Die Trennbedingungen wurden wie in Beispiel 1 gewählt. Die Auftrennung des in Produktreservoirs gewonnenen Heparins zeigte, daß das Molekulargewichtsmaximum des Produkts bei Verwendung der Membran I zu weit im niedermolekularen Bereich lag. Deshalb wurde die Alkoholkonzentration mit Isopropanol schrittweise erhöht und die

0113040

Molekulargewichtsverteilung des Produktes im Produktreservoir durch Hochdruckflüssigkeitschromatographie kontrolliert (N. Sugiska, F.J. Petracek, Fed.Proc. 36 (1), 89 - 92 (1977)).

Tabelle 1 zeigt das Ergebnis. Als Maß für das Molekulargewicht sind die Retentionszeiten der Peakmaxima angegeben. Die Ansatzlösungen enthielten stets 5 % Heparin-Natrium in 0,25 M NaCl. Der pH-Wert lag bei 7,0. Vergleichend wurden zwei weitere Ansätze mit Ethanol getestet.

In Kontrollexperimenten wurde stets überprüft, daß die gefundenen Unterschiede der Retentionszeiten nicht durch einen Lösungsmitteleffekt, der die Säulentrennung beeinflußt, bedingt waren. Dazu wurde das Heparin im Produktreservoir durch Ethanol gefällt, noch einmal umgefällt, der Feststoff gelöst und ebenfalls durch HPLC aufgetrennt. Dabei wurden keine Unterschiede der Retentionszeiten festgestellt.

Tabelle 1

| % Isopropanol | Retentionszeit, Minuten |
| --- | --- |
| - | 6,73 |
| 0,5 | 6,64 |
| 1,0 | 6,53 |
| 2,5 | 6,45 |
| 7,5 | 6,32 |

| % Ethanol | |
| --- | --- |
| 5 | 6,50 |
| 10 | 5,94 |

Für die Herstellung größerer Mengen des gereinigten Heparins wurde für diese Membran eine Isopropanolkonzentration von 7,5 % gewählt.

Beispiel 3

Verwendung der Ultrafiltrationsmembranen der Fa. Amicon H1 P30-43 (I) und H1 P1-43 (II).

Die Ansatzlösung enthielt 250 ml 5 % Heparin-Natriumlösung, pH 7,0 in 250 mM NaCl, 7,5 % Isopropanol (V/V), Temperatur 20°C.

Die Ultrafiltration über die beiden Membranen wurde mit einer Anordnung wie in Beispiel 1 beschrieben durchgeführt. Die "Vorratslösung" enthielt hier jedoch 250 mM NaCl und 7 % Isopropanol (V/V). Aus 12,5 g Heparin-Natrium wurden im Produktreservoir 5,5 g gereinigtes Heparin gefunden. Der Kontrollversuch ohne Isopropanol ergab 2,2 g gereinigtes Heparin. Durch den Diafiltrationsschrittzusatz wurde das Isopropanol restlos entfernt.

Beispiel 4

Durchführung wie in Beispiel 3 beschrieben.
Verwendete Membranen: H1 P30-43 (I), H1 P1-43 (II)

Das Zweimembranverfahren wurde unter Variation der Art und Konzentration des organischen Lösungsmittelzusatzes durchgeführt. Unverändert blieben folgende Parameter:
250 ml 5 % Heparin-Natriumlösung, pH 7,0 in 250 mM NaCl, Temperatur 20°C.

In Tabelle 2 ist als Versuchsergebnis die im Produktreservoir gefundene Menge Heparin-Natrium und die Retentionszeit des Heparinmaximums (HPLC-Methode) angegeben.

Tabelle 2

| Zusatz | | HPLC<br>Retentionszeit,Min. | Heparinmenge<br>g |
|---|---|---|---|
| a.Methanol | 1% | 6,38 | 5,1 |
| | 3% | 6,29 | 5,4 |
| | 5% | 6,25 | 5,5 |
| | 15% | 6,25 | 5,4 |
| b.<br>Ethanol | 1% | 6,66 | 2,4 |
| | 3% | 6,60 | 2,7 |
| | 5% | 6,50 | 3,2 |
| | 15% | 5,94 | 7,0 |
| c.Iso-<br>propanol | 1% | 6,53 | 3,0 |
| | 3% | 6,43 | 3,5 |
| | 5% | 6,40 | 5,1 |
| | 15% | 6,37 | 5,3 |
| d.Kontrolle<br>(ohne Zusatz) | | 6,70 | 2,2 |

Beispiel 5

Durchführung mit gleicher Ansatzmenge wie in Beispiel 3 beschrieben, unter Verwendung zweier Hämodialysatoren:

Membran I:  Secon 103 (B.Braun Melsungen AG)

Membran II: DIACAP (B.Braun Melsungen AG)

Das Membranmaterial dieser Hämodialysatoren besteht aus regenerierter Cellulose.

Es wurden zwei Heparinlösungen eingesetzt:

a) 5 % Heparin-Natrium in 250 mM NaCl, pH 7,0

b. 5 % Heparin-Natrium in 250 mM NaCl, pH 7,0 unter
   Zusatz von 5 % Isopropanol.

Ausbeute: a) 4,3 g
          b) 5,7 g.

### Beispiel 6

Durchführung wie in Beispiel 4 beschrieben, jedoch mit folgender Ansatzlösung:

5 % Heparin-Natrium in 250 mM NaCl, pH 7,0 unter Zuatz von 3 % Acton.

Ausbeute: 6,9 g Heparin.

Gelfiltration des gewonnenen Heparins über eine Sephadex$^R$ G-100-Säule (1,5 x 100 cm) zeigte das gegenüber dem Kontrollversuch höhere mittlere Molekulargewicht.

Elutionsvolumen des gewonnenen Heparins: 110 ml Elutionsvolumen ohne Aceton (Kontrolle): 119 ml

### Beispiel 7

Durchführung mit einer Anordnung wie in Beispiel 1 beschrieben.

Ansatzlösung

250 ml 5 % Heparin-Natrium in 250 mM NaCl, pH 7,0

a) unter Zusatz von 3 % Aceton

b) Kontrolle (ohne Acetonzusatz)

Membran I:   Sartorius SM 14539 (asymetrische Celluloseace-
             tatmembran mit nominaler Molekulargewichts-
             grenze von 10 000)

Membran II:  Hämodialysator DIACAP (B.Braun Melsungen
             AG).

Im Falle a) ließ sich keine Veränderung der Molekular-gewichtsverteilung (Gelfiltration über Sephadex$^R$ G-100) der aus dem Produktreservoir isolierten Substanz gegenüber dem Ausgangsmaterial feststellen. Das Lösungsmittel Aceton hatte offenbar die Membran I geschädigt. Der Kontrollversuch b) ergab ein Elutionsvolumen des Peakmaximums von 105 ml. Das Elutionsvolumen des Peakmaximums des Ausgangsmaterials lag bei 102 ml.

Beispiel 8

50 g Heparin-Natrium wurden in 500 ml $H_2O$ gelöst, über eine Säule mit Kationenaustauscher (Dowex$^R$ 50 W X8, 200 bis 400 mesh) in der $H^+$-Form gegeben (4,5 x 30 cm) und das saure Säuleneluat aufgefangen. Die Heparinsäurelösung wurde am Rotationsverdampfer eingeengt und unter pH-Kontrolle mit Triethylamin bis zu pH 7,0 versetzt. Diese Lösung wurde erneut eingeengt.

Mit dem Heparin-Triethylammoniumsalz wurde folgende Ansatzlösung hergestellt:

1 Liter: 5 % Heparin-Triethylammoniumsalz 250 mM NaCl 35 % Ethanol (w/w).

Diese Lösung wurde unter Verwendung folgender Membranen ultrafiltriert:

Membran I:  Hämodialysator Secon 102

Membran II: Hämodialysator DIACAP

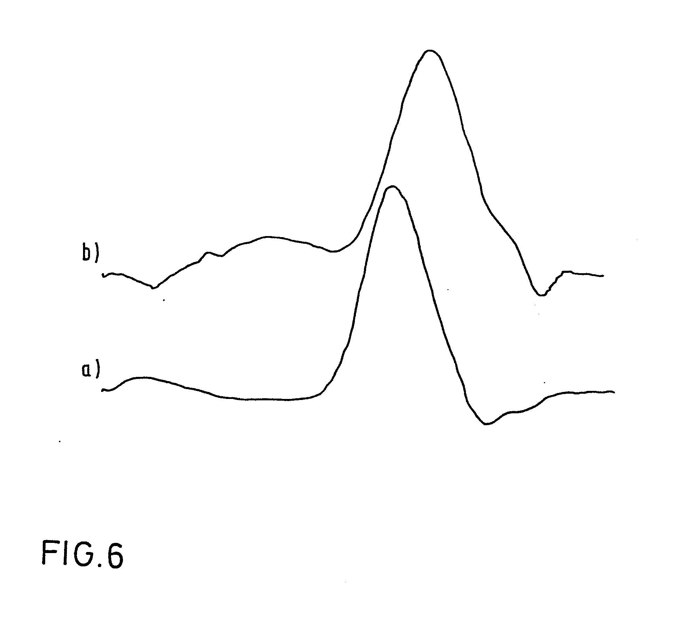

Nach der Ultrafiltration über beide Membranen wurde unter Zugabe von Wasser zum Produktreservoir über die Membran II diafiltriert, bis im Ultrafiltrat II kein Chlorid und kein Ethanol nachweisbar war. Unter Zugabe von festem NaCl wurde eine Endkonzentration von 0,5 M eingestellt und Heparin mit Ethanol (80 %) ausgefällt. Dieser Fällungsschritt wurde nochmals wiederholt. Die Molekulargewichtsverteilung des getrockneten Heparin-Natriums wurde über eine Gelfiltration über eine Sephadex$^R$ G-100-Säule (1,5 x 100 cm) ermittelt (Fig. 6).

Zum Vergleich ist die Molekulargewichtsverteilung von Beispiel 5a (ohne Alkoholzusatz) eingezeichnet.

**Beispiel 9**

Zur Entpyrogenisierung eines pyrogenhaltigen Heparin-Natriumsalzes (Salbenqualität) wurden je 250 ml 5 % Heparinlösung in 250 mM NaCl, pH 7,0, mit einer Anordnung wie in Beispiel 1 beschrieben ultrafiltriert.

Membran II war in jedem Falle eine Hohlfasermembran
(Amicon H1 P1-43)
Membran I  Amicon Hohlfasermembran H1 P10-43
H1 P30-43

Mit jeder Membrananordnung, z.B. H1 P10-43 (Membran I) und H1 P1-43 (Membran II), wurden zwei Versuche mit und ohne Isopropanolzusatz (10 %) durchgeführt. Als Versuchsergebnis wurde die Ausbeute im Produktreservoir und die Anwesenheit von Pyrogenen (Limulus-Test) ermittelt. Tabelle 3 faßt das Ergebnis zusammen:

Tabelle 3

| Membran I | Ausbeute (g) Produktreservoir | | Pyrogentest | |
|---|---|---|---|---|
| | +Isoprop. (10%) | −Isoprop. | +Isoprop. | −Isoprop. |
| H1 P10-43 | 15 | 6 | − | − |
| H1 P30-43 | 46 | 15 | − | − |
| H1 P50-43 | 80 | 40 | − | − |
| H1 P100-43 | 95% | 93% | + | + |

Beispiel 10

8 g Heparin-Natrium bzw. Heparin-Triethylammoniumsalz wurde in 0,25 M NaCl gelöst und, wie in Beispiel 5 beschrieben, ultrafiltriert.

Membran I: Hämodialysator Secon 102
Membran II: Hämodialysator DIACAP

a) 8 g Heparin-Natrium wurde in 0,25 M NaCl gelöst und unter Anwendung des erfindungsgemäßen Zweimembranverfahrens ultrafiltriert.

b) 8 g Heparin-Triethylammoniumsalz (Herstellung gem. Beispiel 8) wurden in 0,25 M NaCl gelöst und, wie bei a) beschrieben, ultrafiltriert.

c) 8 g Heparin-Triethylammoniumsalz wurden in einer Lösung, die 0,25 M Kochsalz und 35 % Ethanol (V/V) enthielt, ultrafiltriert.

Das Heparin in der Ansatzlösung (Retentat) und das in dem Produktreservoir durch die Membran I permeierte Heparin wurden mit Ethanol ausgefällt, in $H_2O$ wieder aufgelöst, nochmals ausgefällt, zentrifugiert und der Niederschlag bei 50°C im Vakuum getrocknet.

Die Molekulargewichtsverteilung der isolierten Heparine wurden hochdruckflüssigkeitschromatographisch ermittelt. Tabelle 4 faßt die Ergebnisse zusammen. Angegeben sind die Retentionszeiten der Peakmaxima (Minuten).

Tabelle 4

| Ansatz | Retentionszeit, Min. | |
| | Retentat | Ultrafiltrat |
| --- | --- | --- |
| a)<br>Heparin-Natrium<br>in 0,25 M NaCl | 5,84 | 6,17 |
| b)<br>Heparin-Triethyl-<br>ammoniumsalz in<br>0,25 M NaCl | 5,83 | 6,18 |
| c) Heparin-Triethyl-<br>ammoniumsalz in<br>0,25 M NaCl und 35 %<br>Ethanol | 5,79 | 5,96 |

Das Ergebnis des Versuches belegt die Verschiebung der Molekulargewichtsverteilung zu höheren Molekulargewichten c) in 35 % alkoholischer Lösung.

0113040

Patentansprüche

1. Verfahren zur Reinigung, Fraktionierung und Entpyrogenisierung von Heparin, seinen Salzen und/oder seinen Derivaten mit Hilfe der Ultrafiltration, dadurch gekennzeichnet, daß man wässrige, Neutralsalz enthaltende Lösungen des Heparins bzw. seiner Salze einem Ultrafiltrations-Zweimembranverfahren unterwirft, wobei Heparin und seine Derivate zunächst in einer ersten Stufe durch eine Ultrafiltrationsmembran I mit höherer molekularer Durchlässigkeit permeieren und erst dann in einer zweiten Stufe von einer Ultrafiltrationsmembran II mit geringerer molekularer Durchlässigkeit zurückgehalten werden.

2. Verfahren nach Anspruch 1, dadurch gekennzeichnet, daß die Membran I eine nominale Molekulargewichtsgrenze von 10 000 bis 50 000, bevorzugt 30 000 bis 50 000 aufweist.

3. Verfahren nach Anspruch 1, dadurch gekennzeichnet, daß die Membran II für Heparinmoleküle undurchlässig ist und eine nominale Molekulargewichtsgrenze von 200 bis 5 000, bevorzugt 1 000, aufweist.

4. Verfahren nach Anspruch 1 - 3, dadurch gekennzeichnet, daß die molekulare Durchlässigkeit der Membran I für Heparin durch Zugabe von wasserlöslichen organischen Lösungsmitteln vergrößert wird.

5. Verfahren nach Anspruch 1 - 4, dadurch gekennzeichnet, daß als organische Lösungsmittel Methanol, Ethanol, Isopropanol oder Aceton oder deren Mischungen in Konzentrationen im Bereich von 0 bis 80 %, vorzugsweise 2 - 15 %, verwendet werden.

6. Verfahren nach Ansprüchen 1 - 5, dadurch gekennzeichnet, daß im pH-Bereich 6 - 8, vorzugsweise 6,5 bis 7,0 gearbeitet wird.

7. Verfahren nach Ansprüchen 1 - 6, dadurch gekennzeichnet, daß als Neutralsalz NaCl oder $CaCl_2$ verwendet wird.

8. Verfahren nach Ansprüchen 1 - 7, dadurch gekennzeichnet, daß die Salzkonzentration zwischen 0,05 und 3 M, bevorzugt bei 0,25 M liegt.

9. Verfahren nach Ansprüchen 1 - 8, dadurch gekennzeichnet, daß Pyrogene aus heparinhaltigen Lösungen abgetrennt werden.

10. Verfahren nach Ansprüchen 1 - 9, dadurch gekennzeichnet, daß Heparine in Form ihrer Natrium- oder Calciumsalze, in Form von Salzen organischer stickstoffhaltiger Ionen, bevorzugt von Monoalkyl-, Dialkyl-, Trialkyl- oder Tetraalkyl-Ammoniumsalzen oder auch von Arylammoniumsalzen oder deren Derivaten, oder in Form von polymeren organischen Ammoniumsalzen, bevorzugt von Salzen des Polyethylenimins, Protamins oder Polylysins, verwendet werden.

11. Verfahren nach Ansprüchen 1 - 10, dadurch gekennzeichnet, daß zunächst der Trennungs- bzw. Reinigungsschritt durch die Membran I erfolgt, sich das durch Membran II zurückgehaltene Heparin in einem Produktreservoir ansammelt und die niedermolekularen Verunreinigungen und das Neutralsalz durch die Membran II unter Wasserzugabe diafiltriert werden.

12. Verfahren nach Anspruch 11, dadurch gekennzeichnet, daß die Diafiltration unter Zuführung von Kochsalzlösung in einer Konzentration erfolgt, daß die Heparinlösung im Produktreservoir eine Gesamtosmolarität von 100 - 500 m Osm aufweist.

13. Verfahren nach Anspruch 11, dadurch gekennzeichnet, daß die Diafiltration unter Zuführung von $CaCl_2$-Lösung bei pH 6,0 bis 8,0 erfolgt.

14. Verfahren nach Ansprüchen 1 - 13, dadurch gekennzeichnet, daß bei Temperaturen von 2 bis 80°C, bevorzugt zwischen 20 und 35°C gearbeitet wird.

15. Verfahren nach Ansprüchen 1 - 14, dadurch gekennzeichnet, daß nach dem Diafiltrationsschritt eine Ausfällung des gereinigten Heparins mit Ethanol mit einer Endkonzentration von 70 bis 90 % (V/V) erfolgt.

16. Verfahren nach Ansprüchen 1 - 14, dadurch gekennzeichnet, daß das in der Ansatzlösung zurückgehaltene, hochmolekulare Heparin durch Ausfällung mit Ethanol mit einer Endkonzentration von 70 - 90 % (V/V) zurückgewonnen wird.

17. Verfahren nach Ansprüchen 1 - 14, dadurch gekennzeichnet, daß heparinhaltige Lösungen mit einer Konzentration von 1 bis 25 %, bevorzugt 4 bis 15 %, eingesetzt werden.

18. Verfahren nach Ansprüchen 1 - 17, dadurch gekennzeichnet, daß andere Glycosaminoglykane als Heparin oder auch synthetische oder halbsynthetische Heparinoide gereinigt werden.

19. Mittel, enthaltend Heparin bzw. seine Derivate, die nach Ansprüchen 1 - 18 hergestellt worden sind.

FIG.1

-1/6-

0113040

0113040

FIG.2

0113040

FIG. 3 a-e

0113040

FIG.4

0113040

FIG.5

b)

a)

FIG.6